# EUROPEAN PATENT APPLICATION

(11) **EP 4 647 007 A1**
(43) Date of publication of application: **12.11.2025**
(21) Application number: 25171328.5
(22) Date of filing: 17.04.2025
(51) Int. Cl.: A61B 5/16, A61B 5/00

(54) **METHODS AND SYSTEMS FOR GENERATING AND UTILIZING DATA FOR DETECTION AND CONTINUOUS MONITORING OF HUMAN STATES**

(30) Priority: 07.05.2024 US 202418657604
(71) Applicant: Harman International Industries, Inc., Stamford, Connecticut 06901 (US)
(72) Inventor: FILIMONOV, Andrey Viktorovich, Stamford, CT, 06901 (US); SHISHALOV, Ivan Sergeevich, Stamford, CT, 06901 (US); BAKHCHINA, Anastasiya Vladimirovna, Stamford, CT, 06901 (US); ARUTYUNOVA, Karina, Stamford, CT, 06901 (US); IVASHKOV, Denis Viktorovich, Stamford, CT, 06901 (US); KONOVALOV, Daniil Igorevich, Stamford, CT, 06901 (US); TIMAKIN, Nikita, Stamford, CT, 06901 (US); BURASHNIKOV, Evgenii Pavlovich, Stamford, CT, 06901 (US); MARGARYAN, Mane, Stamford, CT, 06901 (US); KLESHNIN, Mikhail Sergeevich, Stamford, CT, 06901 (US)
(74) Representative: Westphal, Mussgnug & Partner, Patentanwälte mbB

(57) **Abstract**

The present disclosure relates to methods and systems for establishing ground truth in human state detection. In one embodiment, the disclosure teaches recording physiological data over a continuous duration of at least a threshold duration that is sufficient for capturing representation of the target state in bio signals used for detection. The recorded data is segmented into shorter analysis windows, as a step toward continuous state detection, each window less than the threshold duration, and labeled with a ground truth indicative of the target state. The windows and labels are stored in non-transitory memory, for later use in training, testing, and validating one or more state prediction models. The method enhances the accuracy of state detection models by providing a more representative ground truth through prolonged recordings, which capture the variable manifestations of human states in physiological processes.

## Description

### TECHNICAL FIELD

The present disclosure relates generally to the field of human state detection and monitoring. More specifically, the disclosure pertains to generating and utilising ground truth data for training, testing, and validating systems for continuous monitoring of the human state.

### BACKGROUND

In recent years, the development of solutions for detecting and continuous monitoring human states, such as drowsiness, stress, and cognitive load, has garnered significant interest, particularly in activities where performance and attention are relevant factors, such as driving. Current methods for human state detection rely on either subjective or objective measures of underlying psychophysiological states, often utilizing physiological signals recorded from the heart, brain, skin, eyes, and other organs. These signals are reflective of the underlying psychophysiological states that can influence, and be influenced by, an individual's behavior and performance.

Despite advancements in mathematical modeling and machine learning/artificial intelligence (ML/AI) capabilities, the accurate detection of human states remains a challenging endeavor. The inventors have identified that one of the primary difficulties for accurate prediction of human states lies in the dynamic and variable nature of physiological manifestations of underlying psychophysiological states, e.g., manifestations of human states may present sporadically, and may be interspersed with various behaviors and mental activities relevant to the subject but not always directly related to current predominate psychophysiological state. One consequence of such variable presentation in human state expression is that the recordings of objective data used to train, test and validate state prediction models may not consistently capture the full range of manifestations representative of an underlying psychophysiological state if the recordings are not long enough. At the same time, continuous monitoring requires more granular time resolution which often necessitates giving prediction for intervals of time shorter than ground truth required to capture physiological dynamics truly representative of the target state. Thus, human state prediction models using datasets with short windows as ground truth may be inaccurate, or display inconsistent sensitivity to different manifestations of a same underlying human state (e.g., a model may be highly sensitive to a first subset of manifestations, and insensitive to a second subset of manifestations, of a same psychophysiological state); while models using longer windows may not be suitable for continuous detection and monitoring.

The complexity of human state detection is further compounded by the fact that no psychophysiological state is entirely monolithic. For instance, the state of high cognitive load may be sub-divided into aspects related to learning, decision-making, response, reflection, frustration, and more. Each of these distinct aspects of a particular psychophysiological state may have variable effects on physiological processes and are not uniformly distributed over time, even if a subject may be considered as uniformly experiencing a target state. Similarly, the observable levels and physiological manifestations of drowsiness may fluctuate, particularly in the presence of microsleep episodes, which poses additional challenges for state prediction models that rely on data derived from short physiological recordings; alternatively, longer windows may not provide sufficient time resolution required to take any action if, for example, a subject is falling asleep while driving.

Moreover, the collection and analysis of physiological data for the purpose of state detection has constraints in terms of efficiency and resource utilization. The recruitment of participants and the modeling of conditions to elicit target states are resource-intensive endeavors. The limited availability of large datasets for modeling correlations between physiological manifestations and underlying psychophysiological states is a generally recognized bottleneck in the field of human state detection, and is one roadblock to training robust and highly accurate human state prediction models.

In light of these challenges, there is a need for improved approaches for efficiently generating and using robust datasets for human state prediction that account for the dynamic and variable nature of physiological manifestations of underlying human states. Such approaches enable more accurate and efficient data collection, processing, and analysis, thereby enabling the training and validating of continuous state prediction models and monitoring systems. The present disclosure seeks to address these and other related technical issues within the field of human state detection.

### SUMMARY

The current disclosure at least partially addresses the issues described above. In one aspect, a method is provided for recording and analyzing physiological data to evaluate a target state in a subject. The method includes recording physiological data from a subject when the subject is in a target state for a continuous duration that is equal to or greater than a threshold duration selected as a sufficient representation of the target state in recorded bio signals. The recorded physiological data is then segmented into a plurality of shorter analysis windows, with each analysis window having a pre-determined duration that is less than the threshold duration, and the plurality of shorter analysis windows is labeled with a ground truth label indicative of the target state, as a step toward continuous state detection. The method further includes storing the plurality of shorter analysis windows and the associated ground truth label in non-transitory memory.

In another aspect, a system is disclosed for inducing and evaluating a target state in a subject. The system includes a processor and a non-transitory memory storing instructions that, when executed by the processor, cause the system to induce the target state in the subject. The system records physiological data from the subject over a continuous duration equal to or greater than a threshold duration. The recorded physiological data is segmented into a plurality of shorter analysis windows, each analysis window being of a pre-determined duration of equal to or less than the threshold duration. The system labels each of the plurality of shorter analysis windows with a ground truth label indicative of the target state and stores the plurality of shorter analysis windows and the ground truth label in the non-transitory memory.

In yet another aspect, a method is provided for training a state prediction model. The method includes inducing a target state in a subject and recording physiological data from the subject over a continuous duration greater than or equal to a threshold duration. The recorded physiological data is segmented into a plurality of physiological data windows, each of the plurality of physiological data windows being of a pre-determined duration of less than the threshold duration. Each of the plurality of physiological data windows is labeled with a ground truth label indicative of the target state. The method includes storing the plurality of physiological data windows and the ground truth label in non-transitory memory. A training data pair is selected, comprising the plurality of physiological data windows of pre-determined duration, and the ground truth label indicative of the target state. The method involves mapping the plurality of physiological data windows to a corresponding plurality of state predictions using the state prediction model. A loss for the plurality of state predictions is determined based on a loss function and the ground truth label. Parameters of the state prediction model are updated based on the determined loss.

The disclosed methods and systems at least partially address the challenges identified above by leveraging extended-duration physiological recordings to establish a more representative ground truth for human states. This approach acknowledges the dynamic and variable nature of physiological manifestations, which may not be adequately captured by shorter conventional recordings. By recording physiological data over a continuous duration of at least the threshold duration, the methods and systems capture a broader spectrum of physiological responses, thereby mitigating the risk of inaccuracies caused by the sporadic and interspersed nature of human state expressions. The subsequent segmentation of these extended recordings into shorter analysis windows, each less than the threshold duration, allows for the continuous monitoring of the subject's state with enhanced temporal resolution. This segmentation not only reflects the variable manifestations of states such as cognitive load and drowsiness but also aligns with the need for high-frequency state detection in certain applications, including driver alertness monitoring. Furthermore, the disclosed embodiments significantly improve the efficiency of data collection and processing. By generating a larger dataset from prolonged recordings, which can be subdivided into numerous shorter windows, the methods and systems improve the efficiency of resources utilization. The expanded datasets enabled by the current disclosure provide a rich foundation for the application of machine learning and artificial intelligence algorithms, facilitating the development of more robust and accurate models for real-time human state detection.

### BRIEF DESCRIPTION OF THE DRAWINGS

Various aspects of the present disclosure may be better understood upon reading the following detailed description and upon reference to the drawings in which:
FIG. 1 is a schematic representation of a process for training a human state prediction model;
FIG. 2 is a block diagram of a human state prediction model training system;
FIG. 3 is a flowchart of a method for generating training data for a human state prediction model;
FIG. 4 is a flowchart of a method for detecting and labeling state manifestations;
FIG. 5 is a graphical illustration contrasting physiological manifestations of low and high cognitive load conditions;
FIG. 6 is a flowchart of a method for training a human state prediction model;
FIG. 7 is a flowchart depicting a method for evaluating the discriminatory power of a trained human state prediction model by comparing state predictions across different induced states;
FIG. 8 is a graphical illustration of two distinct methods of comparison between human states; and
FIG. 9 is a flowchart of a method for determining an aggregate performance metric for a mathematical state prediction model.

### DETAILED DESCRIPTION

The present disclosure relates to methods and systems for generating and utilizing physiological data in human state detection, particularly for continuous monitoring of human states over selected time windows. The methods and systems disclosed herein provide for inducing a target psychophysiological state in a subject, recording physiological data over a continuous duration that is sufficient for capturing representation of the target state in recorded bio signals, segmenting the recorded data into shorter analysis windows as a step toward continuous state detection, labeling each window with a ground truth label indicative of the target state, and storing the windows and labels in non-transitory memory.

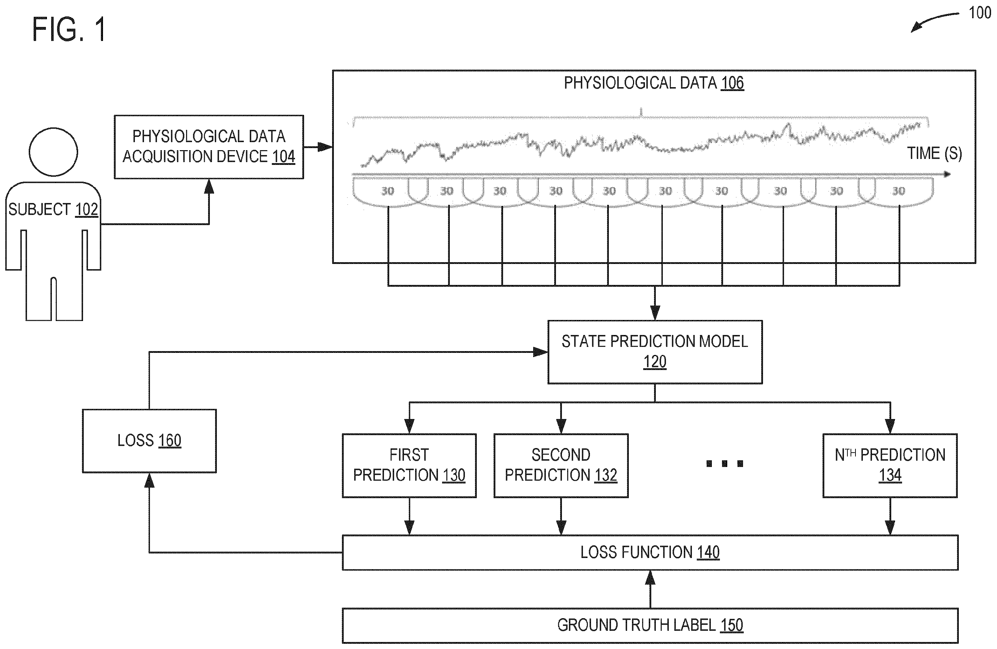

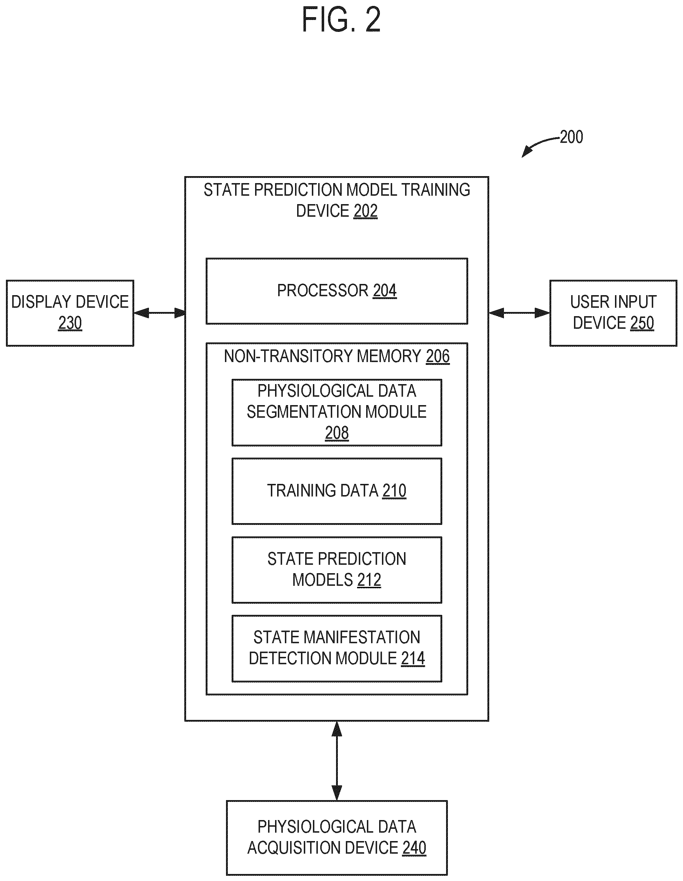

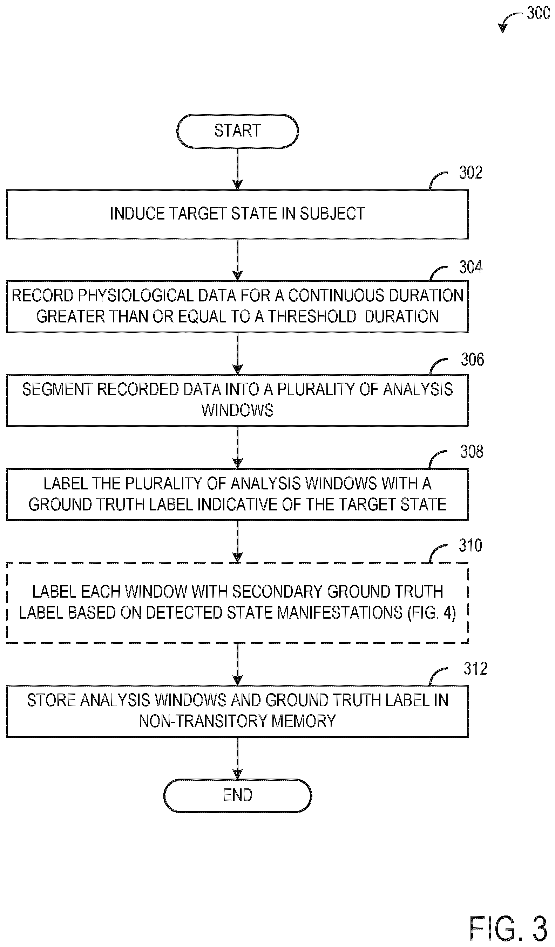

In one embodiment, a process 100 for generating ground truth data and training a state prediction model is depicted in FIG. 1. The process 100 captures and utilizes prolonged physiological recordings to establish a ground truth for various human states, such as cognitive load and drowsiness, which can be used in mathematical modelling and to train machine learning (ML) and artificial intelligence (AI) models for accurate state prediction. The process 100 may be performed by a human state prediction model training system 200, as further detailed in FIG. 2, by executing one or more operations of method 300, shown in FIG. 3. Method 300 includes collecting and labeling physiological data over extended periods and processing this data into shorter analysis windows, which ensures that the training data encompasses a wide range of human state manifestations, thereby enhancing the model's ability to detect human states with higher accuracy.

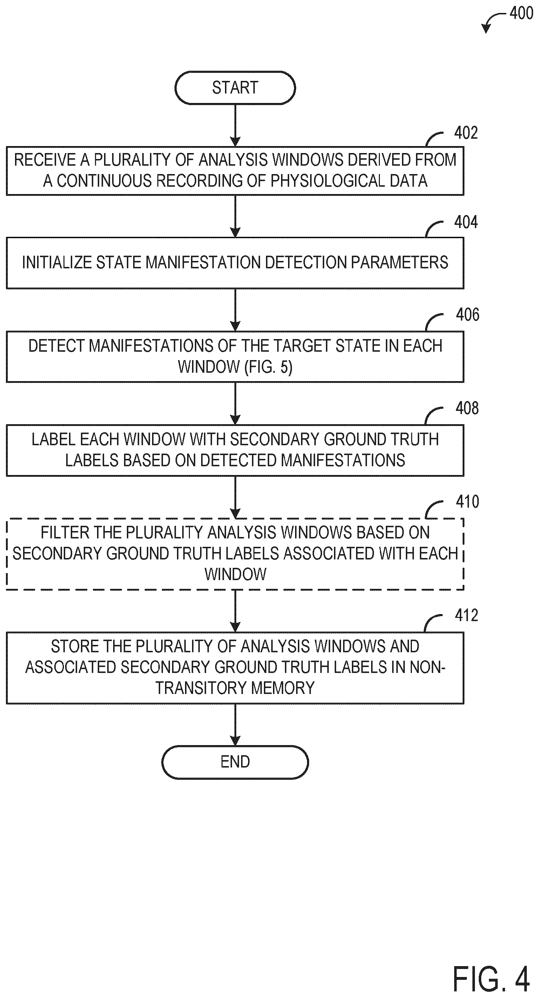

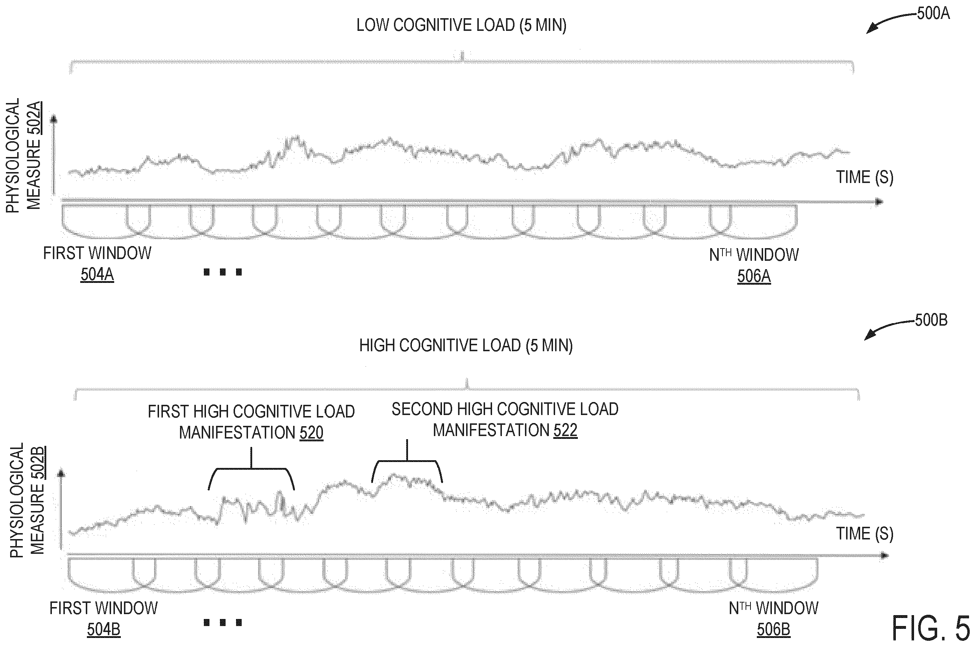

FIG. 4 illustrates a flowchart of a method 400 for detecting and labeling human state manifestations, which may be used for model training, or filtering of analysis windows prior to training, as well as for testing, validating, and comparing models. The method involves identifying specific physiological markers that correlate with different states, such as cognitive load or drowsiness, during various activities, including driving simulations. A graphical illustration contrasting physiological measures under low and high cognitive load conditions is provided in FIG. 5. The illustration of FIG. 5 highlights the variability of physiological measures/manifestations even when a subject is uniformly experiencing one human state.

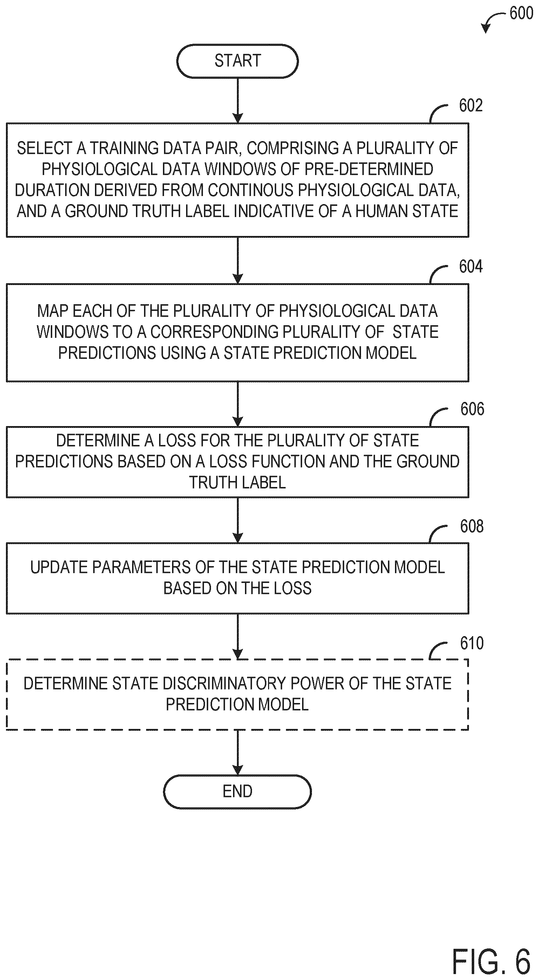

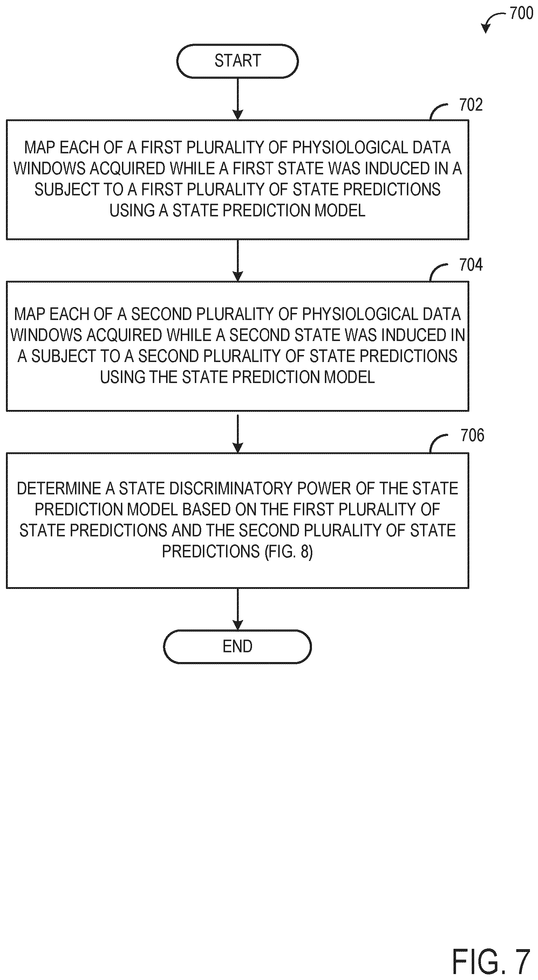

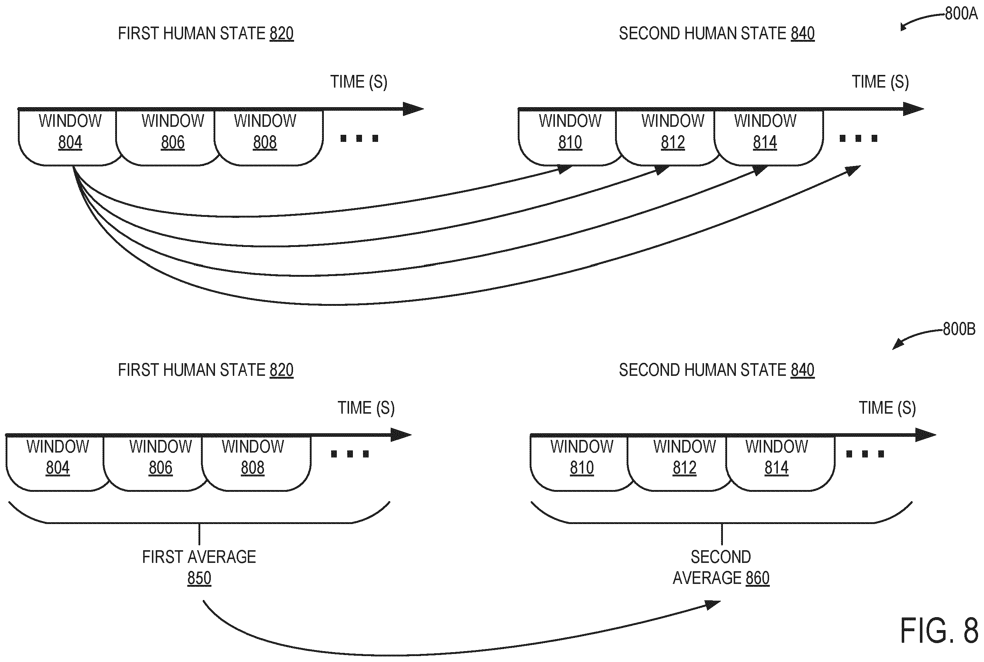

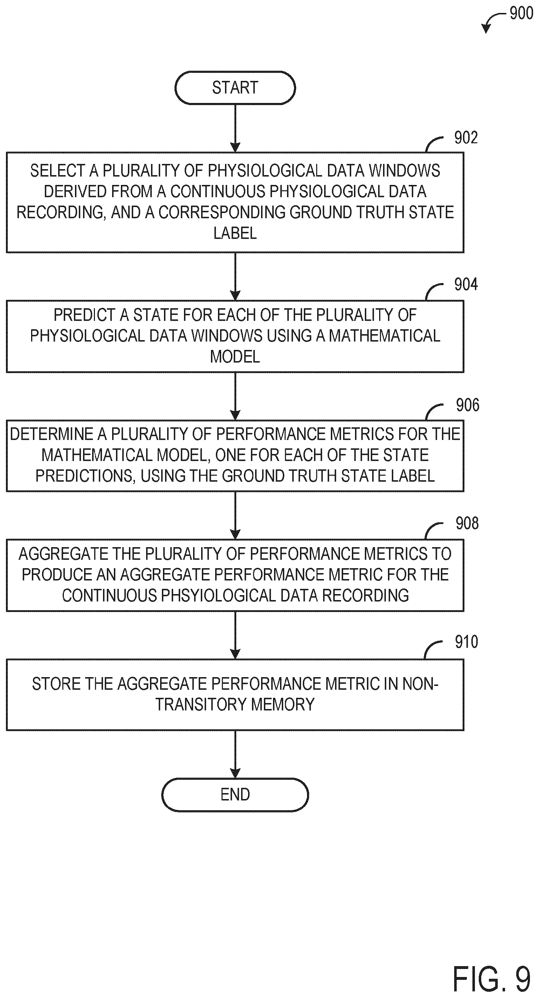

The training of the human state prediction model is depicted in FIG. 6, which presents a flowchart of a method 600 of one embodiment for training an ML model to predict a human state of a subject based on one or more physiological data windows. FIG. 7 provides a flowchart depicting a method 700 for evaluating the discriminatory power of a trained state prediction model. This evaluation method compares state predictions across different induced states to assess the model's accuracy and sensitivity to state variations. FIG. 8 offers a graphical illustration of two distinct methods of comparison between human states, such as may be used in method 700 to evaluate a trained state prediction model's ability to differentiate between a first and second human state. Similarly, an aggregate performance metric of a mathematical human state prediction model, which may in some embodiments comprise a machine learning model, may be determined using the method 900 shown in FIG. 9.

Together, the disclosed systems and methods provide a novel approach to human state detection and continuous monitoring, leveraging longer periods of physiological data to train more robust and accurate prediction models. This approach has significant implications for enhancing the accuracy and performance of continuous state monitoring systems, particularly in scenarios such as vehicle operation or high-stress work environments.

Referring to FIG. 1, a human state prediction training process 100 is depicted, illustrating an embodiment of a process for generating physiological data and utilizing this data to develop a robust human state prediction model. The process 100 may be particularly advantageous in applications such as continuous monitoring of human states, where accurate prediction of human states based on physiological data is necessary.

The subject 102 represents an individual from whom physiological data is acquired during the process 100. In one embodiment, the subject 102 may be a participant in a study designed to elicit a target human state, such as cognitive load or drowsiness, particularly in scenarios where continuous monitoring is desired, such as during driving simulations. The subject 102 may be exposed to various tasks or conditions intended to induce the target state, and the physiological responses are recorded over a continuous duration.

The physiological data acquisition device 104 is configured to record physiological data from the subject 102. In one embodiment, the physiological data acquisition device 104 may include a combination of sensors and instruments, such as electrocardiogram (ECG) monitors, electroencephalogram (EEG) systems, photoplethysmogram (PPG) sensors, skin conductance sensors, and eye gaze tracking devices. These devices are employed to capture a comprehensive set of physiological parameters that are indicative of the subject 102's human state over a continuous duration of at least a threshold duration (e.g., five minutes).

The physiological data 106 comprises one or more measures of physiological parameters recorded over a duration greater than the threshold duration (e.g., 5 minutes). While in this example the duration is substantially longer than a few seconds, the threshold duration may be adjusted based on an indication of a subject physiological parameters, such as breathing rate, activity level, etc. To transform the physiological data 106 into more usable data sets, the data is segmented into a plurality of shorter analysis windows, each with a pre-determined duration that is shorter than the first threshold duration (e.g., 30 seconds, although 30 seconds may be used as an example of the pre-determined duration for the plurality of shorter analysis windows herein, it will be appreciated that durations greater or shorter than 30 seconds may be used, so long as the duration of the shorter analysis windows is less than the threshold duration of the recorded physiological data). This segmentation allows for the continuous monitoring of the subject 102's state with enhanced temporal resolution. In one embodiment, the segmentation may involve overlapping consecutive analysis windows by a predetermined step duration of less than the pre-determined duration of the analysis windows, thereby ensuring a comprehensive representation of the human state over time.

The human state prediction model 120 is a computational model designed to predict the human state of the subject 102 based on the physiological data 106. In one embodiment, the human state prediction model 120 may utilize ML or AI algorithms that are trained using the physiological data 106. The model 120 may be configured to map the plurality of physiological data windows to corresponding human state predictions, such as the first prediction 130, the second prediction 132, and the N^{th} prediction 134, where N is a positive integer greater than two.

The first prediction 130, the second prediction 132, and the N^{th} prediction 134 represent a series of human state predictions generated by the human state prediction model 120 for each of the shorter analysis windows derived from the physiological data 106. In various embodiments, these predictions may be binary, categorical, or probabilistic in nature, reflecting the likelihood or presence of the target human state within each analysis window.

The loss function 140 is a mathematical construct used to quantify the error or loss between the predictions generated by the human state prediction model 120 and the ground truth label 150. In one embodiment, the loss function 140 may be a mean squared error function, a cross-entropy loss function, or any other suitable loss function known in the art of machine learning. The loss function 140 enables the training and optimization of the human state prediction model 120 by determining the adjustment of the model's parameters during the training process.

The ground truth label 150 is assigned to the entire duration of the physiological data 106 and, by extension, to each of the plurality of shorter analysis windows. In one embodiment, the ground truth label 150 may be indicative of the target human state that the subject 102 was intended to experience during the data acquisition phase. The ground truth label 150 serves as a reference against which the predictions of the human state prediction model 120 are compared.

The Loss 160 is determined based on the difference between the predictions (first prediction 130 through to N^{th} prediction 134) and the single ground truth label 150 for the physiological data 106. In one embodiment, the loss 160 may be calculated based on a difference between an average prediction (made across the ground truth time scale) and the ground truth label 150, rather than comparing individual predictions directly with ground truth label 150. This approach acknowledges the dynamic and variable nature of physiological manifestations in physiological measures and aims to provide a more accurate assessment of the human state prediction model's 120 performance.

In alternative embodiments, the human state prediction training data generation process 100 may include additional components or operations, such as filtering the plurality of shorter analysis windows based on secondary ground truth labels indicative of manifestations of the target human state, or utilizing different window sizes and step durations to optimize the temporal resolution and accuracy of the human state predictions.

Together, the components and operations of the human state prediction training process 100 provide a comprehensive approach to generating training data for the development of accurate and robust models for real-time human state detection.

Referring to FIG. 2, a human state prediction model training system 200 for training models to predict human states from physiological data is shown. The system 200 is configured to enhance the capabilities of continuous monitoring applications by leveraging physiological data to provide accurate, relevant, and contextually appropriate predictions of human states such as cognitive load and drowsiness. The system 200 includes a state prediction model training device 202, which is designed to interact with various components and display results via a display device 230.

The state prediction model training device 202 comprises a processor 204, which is configured to execute machine-readable instructions stored in a non-transitory memory 206. The processor 204 may be a single-core or multi-core processor, and the programs executed thereon may be configured for parallel or distributed processing. In some embodiments, the processor 204 may include individual components that are distributed throughout two or more devices, which may be remotely located and/or configured for coordinated processing. In other embodiments, aspects of the processor 204 may be virtualized and executed by remotely-accessible networked computing devices configured in a cloud computing configuration.

The non-transitory memory 206 stores machine-readable instructions that, when executed by the processor 204, enable the device 202 to perform various functions related to human state prediction model training. Within the non-transitory memory 206, a physiological data segmentation module 208 is stored. The physiological data segmentation module 208 is trained to segment physiological data into a plurality of shorter analysis windows, each window being of a pre-determined duration of less than the threshold duration (e.g., five minutes). In one embodiment, the physiological data segmentation module 208 may utilize an algorithm to segment the data with overlapping consecutive analysis windows by a predetermined step duration of less than the pre-determined duration of the analysis windows, thereby expanding the data volumes and variety of possible data representations for training and testing the continuous state prediction models.

Also stored within the non-transitory memory 206 is training data 210. The training data 210 stores a plurality of physiological data windows, each uniquely associated with a corresponding ground truth label indicative of the target human state. In one embodiment, the training data 210 may be used to train one or more of the state prediction models 212 by executing one or more operations of method 600.

The state prediction models 212, which are also stored within the non-transitory memory 206, are configured to map the plurality of physiological data windows to a corresponding plurality of human state predictions. In some embodiments, the state prediction models 212 may be ML models configured to generate human state predictions based on physiological data such as ECG data, EEG data, PPG data, skin conductance data, and eye gaze data.

A state manifestation detection module 214 is included within the non-transitory memory 206. The state manifestation detection module 214 is configured to detect in each of the plurality of shorter analysis windows the occurrence of one or more of a pre-determined set of manifestations of the target human state. In one embodiment, the state manifestation detection module 214 may employ machine learning techniques to identify specific physiological markers that correlate with different states, such as cognitive load or drowsiness, during various activities, including driving simulations.

The user input device 250 is configured to interface with the human state prediction model training device 202. The user input device 250 may be a computer, a smartphone, a tablet, or any other device capable of submitting user input to the system 200 and receiving responses. The user input device 250 may include a user interface that allows users to interact with the system 200, input commands, and view the results generated by the state prediction models 212 based on the data retrieved from the training data 210.

The display device 230 is communicably coupled to the processor 204 and is configured to display results and information related to the human state prediction model training. The display device 230 may include one or more display devices utilizing virtually any type of technology, such as a computer monitor, a touchscreen, or a projector. In some embodiments, the display device 230 may be combined with the processor 204 and non-transitory memory 206 in a shared enclosure, or it may be a peripheral display device.

The physiological data acquisition device 240 is configured for capturing physiological data from subjects over a continuous duration of at least the threshold duration (e.g. five minutes). The physiological data acquisition device 240 may include a variety of sensors and measurement tools such as ECG monitors, EEG headsets, PPG sensors, skin conductance sensors, and eye-tracking devices. In one embodiment, the physiological data acquisition device 240 may be configured to record data in a simulated driving task environment to induce a target human state in the subject. In another embodiment, the device 240 may be adapted to capture physiological data in a variety of settings, including clinical environments or during the performance of cognitive tasks.

In alternative embodiments, the components of the state prediction model training device 202 may include additional modules or features to enhance the system's capabilities. For example, the system 200 may be adapted to support multiple human states, making it versatile for a range of applications from driver alertness monitoring to stress and fatigue detection in high-stress work environments.

Referring to FIG. 3, a flowchart of a method 300 for generating human training data is shown. The method 300 may be employed by a system, such as human state prediction model training system 200, to train continuous human state prediction models with enhanced accuracy and robustness.

At operation 302, the system induces a target human state in a subject. The induction of the target state may be achieved through various experimental setups designed to elicit specific human responses. In one embodiment, the system administers a cognitive task to the subject, such as an n-back task, a simulated driving task, or a pattern recognition task, to induce the target human state. In another embodiment, the system may moderate the cognitive task to adjust the level of cognitive load experienced by the subject. This moderation may involve varying the complexity of the cognitive task, the frequency of task stimuli, and the duration for which the cognitive task is performed by the subject. In a further embodiment, the system may employ a combination of sensory stimuli, such as auditory or visual cues, to induce the target human state, thereby simulating real-world conditions that may affect the subject's state, such as driving or operating machinery.

Proceeding to operation 304, the system records physiological data from the subject over a continuous duration of at least the threshold duration (five minutes). The recording of physiological data over this extended duration enables capturing a more complete range of manifestations of the target human state. In one embodiment, the system utilizes a suite of physiological data acquisition devices configured to measure heart rate, skin conductance, brain activity, and respiratory rate. In another embodiment, the physiological data includes at least one of ECG data, EEG data, PPG data, skin conductance data, and eye gaze data. In a further embodiment, the system may employ wearable sensors that allow for the unobtrusive collection of physiological data while the subject performs the cognitive task, thereby minimizing any potential interference with the subject's natural human responses.

At operation 306, the system segments the recorded physiological data into a plurality of shorter analysis windows. Each analysis window is of a pre-determined duration of less than the threshold duration, allowing for the continuous monitoring of the subject's state with enhanced temporal resolution. In one embodiment, the pre-determined duration of the plurality of shorter analysis windows is 30 seconds, which aligns with the need for high-frequency state detection in certain applications. In another embodiment, the system segments the recorded physiological data into the plurality of shorter analysis windows by overlapping consecutive analysis windows by a predetermined step duration of less than the pre-determined duration of the analysis windows. This overlapping ensures that the variable manifestations of the human state are captured more comprehensively. In a further embodiment, the system may utilize advanced signal processing techniques to ensure that the segmentation of the physiological data preserves the integrity of the physiological signals, thereby maintaining the quality of the data for subsequent analysis.

Transitioning to operation 308, the system undertakes the task of assigning a ground truth label to each of the segmented shorter analysis windows, with each label being reflective of the target human state induced in the subject at operation 302. In one embodiment, the ground truth label for each window is derived directly from the induced target human state, without the need for complex inferential processes or assumptions. This direct derivation ensures that the label accurately represents the human state that the subject was intended to experience during the data acquisition phase. The labeling process establishes a reliable reference against which the performance of human state prediction models can be evaluated.

At operation 310, the system labels each window with a secondary ground truth label based on detected human state manifestations, as further detailed in FIG. 4. This operation involves identifying specific physiological markers that correlate with different states. In one embodiment, the system detects in each of the plurality of shorter analysis windows the occurrence of one or more of a pre-determined set of manifestations of the target human state. In another embodiment, the system labels each of the plurality of shorter analysis windows with a secondary ground truth label indicative of the detected manifestations of the target human state. In a further embodiment, the system may filter the plurality of shorter analysis windows based on a plurality of respective secondary ground truth labels indicative of manifestations of the target human state, thereby refining the dataset for model training.

Finally, at operation 312, the system stores the analysis windows and the ground truth label in non-transitory memory. The storage of the labeled analysis windows in non-transitory memory facilitates the later retrieval and utilization of the data for training and validating human state prediction models. In one embodiment, the system organizes the stored data in a structured database, allowing for efficient querying and access to the analysis windows and associated labels. In another embodiment, the system may employ data encryption and access control mechanisms to ensure the privacy and security of the stored physiological data. Following operation 312, method 300 may end.

In this way, method 300 enables the generation of physiological data that is representative of the dynamic and variable nature of human states. By leveraging extended-duration physiological recordings, the system provides a rich dataset for the application of machine learning and artificial intelligence algorithms, their testing, validation and comparison, ultimately leading to the development of more robust and accurate models for real-time human state detection.

Referring to FIG. 4, a flowchart of a method 400 for detecting manifestations of a target human state in a continuous recording of physiological data is shown. The method 400 enables the identification and labeling of human state manifestations within analysis windows derived from a continuous recording, facilitating the establishment of a ground truth for training and validating human state prediction models. The method 400 utilizes a series of operations to process the physiological data, detect state manifestations, assign secondary ground truth labels, and store the processed data for subsequent use.

At operation 402, the method 400 begins with the receipt of a plurality of analysis windows derived from a continuous recording of physiological data. These analysis windows are segments of physiological data that have been previously recorded over a continuous duration of at least the threshold duration. In one embodiment, the physiological data may include, but is not limited to, ECG data, EEG data, PPG data, skin conductance data, and eye gaze data. Each analysis window is of a pre-determined duration, such as 30 seconds, and is designed to capture the dynamic and variable nature of physiological manifestations of the target human state. In another embodiment, the analysis windows may be overlapped by a predetermined step duration to ensure a comprehensive representation of the human state over time.

Proceeding to operation 404, the method involves initializing human state manifestation detection parameters. These parameters are employed in the detection of state manifestations within each analysis window. In one embodiment, the initialization may involve setting thresholds for physiological signal variability, patterns indicative of the target state, and other criteria based on the physiological data types being analyzed. For instance, parameters for ECG data might include heart rate variability thresholds, while parameters for EEG data might focus on specific brainwave patterns associated with cognitive load or drowsiness. In another embodiment, the initialization of detection parameters may involve calibrating the system based on a training dataset that includes labeled examples of the target human state manifestations.

At operation 406, the method includes detecting manifestations of the target human state in each window. In one embodiment, the detection may involve applying machine learning or artificial intelligence algorithms that have been trained to recognize patterns in the physiological data that correlate with the target state. For example, a machine learning model may analyze ECG and EEG data to detect signs of cognitive load during a simulated driving task. In another embodiment, the detection may involve statistical analysis of the physiological signals to identify deviations from baseline measures that are indicative of the target state, such as increased skin conductance in response to stress.

Following the detection of state manifestations, operation 408 involves labeling each window with secondary ground truth labels based on the detected manifestations. In one embodiment, the labeling may be binary, indicating the presence or absence of a particular manifestation of a pre-determined set of manifestations of the target human state within the window. In another embodiment, the labeling may be categorical, reflecting different levels or intensities of each of a plurality of manifestations in the pre-determined set of manifestations.

At operation 410, the method 400 optionally filters the plurality of analysis windows based on secondary ground truth labels associated with each window. This filtering process ensures that only the most representative windows are retained for further analysis and model training. In one embodiment, windows with labels indicating a high confidence in the presence of the target state may be selected, while those with lower confidence may be excluded. In another embodiment, the filtering may involve selecting windows that exhibit a range of manifestations of the target state to ensure that the prediction model can generalize across different expressions of the state.

Finally, at operation 412, the method stores the plurality of analysis windows and associated secondary ground truth labels in non-transitory memory. This storage facilitates the accessibility and retrieval of the processed data for future use in training and validating human state prediction models. In one embodiment, the data may be organized in a structured database, allowing for efficient querying based on various criteria, such as the type of physiological data or the intensity of the target state. In another embodiment, the data may be encrypted and access-controlled to ensure the privacy and security of the information. Following operation 412, method 400 may end.

Referring to FIG. 5, a comparison between physiological data under varying cognitive load conditions is depicted. The graphical representation provides a visual comparison between physiological data under low cognitive load 500A and physiological data under high cognitive load 500B. This comparison illustrates the dynamic and variable nature of physiological processes, as they relate to cognitive load, and how distinguishing between distinct human states may rely on a small number of manifestations of the underlying physiological randomly distributed in time.

Physiological data under low cognitive load 500A is characterized by a set of physiological measures 502A, which may include, but are not limited to, heart rate, ECG data, EEG data, PPG data, skin conductance data, and eye gaze data. These measures are indicative of the subj ect's human state when subj ected to minimal cognitive demands. In one embodiment, the physiological measure 502A may represent a baseline or control condition, where the subject is at rest or engaged in a task that requires minimal cognitive effort. In another embodiment, the physiological measure 502A may be obtained during a period of passive observation or during a simple repetitive task that does not significantly engage cognitive resources.

The first window 504A through to the N^{th} window 506A represent discrete segments of the continuous physiological data recorded over a threshold duration, such as five minutes or longer. While the remaining description illustrates example operation with a threshold of 5 minutes as the threshold duration, it should be appreciated that other threshold durations may be used, as explained herein. Each window, such as the first window 504A, is of a pre-determined duration, for example, 30 seconds, and is labeled with a ground truth label indicative of the low cognitive load state. The N^{th} window 506A signifies the last in a series of such windows. In one embodiment, consecutive analysis windows may overlap by a predetermined step duration, such as one second, to ensure a comprehensive representation of the physiological data. In another embodiment, the windows may be non-overlapping, providing distinct snapshots of the physiological state at different time intervals.

Physiological data under high cognitive load 500B is similarly characterized by physiological measures 502B, which are recorded when the subject is subjected to tasks designed to elicit a high cognitive load. These tasks may include, but are not limited to, an n-back task, a simulated driving task with additional cognitive demands, or a complex pattern recognition task. The physiological measure 502B captures the heightened human responses associated with increased cognitive effort, such as changes in heart rate variability, EEG patterns indicative of heightened mental engagement, or changed frequency of eye blinks and saccades.

The first high cognitive load manifestation 520 and the second high cognitive load manifestation 522 represent specific features or patterns within the physiological data that are indicative of high cognitive load. For example, the first high cognitive load manifestation 520 may correspond to a particular pattern of heart rate variability that is known to be associated with intense cognitive processing. The second high cognitive load manifestation 522 may represent a distinct EEG waveform pattern, such as increased theta wave activity, which is commonly linked to tasks requiring significant attention and working memory resources. These manifestations are detected within each of the shorter analysis windows, such as the first window 504B and the N^{th} window 506B, and are labeled with secondary ground truth labels indicative of the detected manifestations of the high cognitive load state.

The first window 504B and the N^{th} window 506B under high cognitive load conditions are analogous to the windows described under low cognitive load conditions, with each window representing a segment of the continuous physiological data. However, these windows are expected to exhibit variations in the physiological measures that reflect the increased cognitive demands placed on the subject. In one embodiment, the windows under high cognitive load conditions may show greater variability in physiological measures compared to the low cognitive load condition, as the subject's human state fluctuates in response to the cognitive tasks. In another embodiment, the windows may reveal patterns of physiological response that correlate with specific stages or subprocesses involved in the cognitive task, such as the onset of a decision-making process or the period of reflection following a task response.

Referring to FIG. 6, a flowchart of a method 600 for training a human state prediction model using physiological data is shown. The method 600 is configured to enhance the capabilities of continuous monitoring applications by leveraging physiological data to provide accurate predictions of human states such as cognitive load and drowsiness.

At operation 602, the system selects a training data pair comprising a plurality of physiological data windows and associated ground truth labels indicative of a target human state. In one embodiment, the physiological data windows may be derived from continuous recordings of physiological data, such as electrocardiogram (ECG) data, EEG data, PPG data, skin conductance data, and eye gaze data, recorded over a duration of at least five minutes. The ground truth labels are assigned based on the target human state that the subject was intended to experience during the data acquisition phase. In another embodiment, the training data pair may be filtered based on secondary ground truth labels associated with detected manifestations of the target human state, ensuring that the model is trained on data that accurately represents the state's variable manifestations.

Proceeding to operation 604, the system maps the plurality of physiological data windows to corresponding human state predictions using the human state prediction model. This mapping process involves applying the prediction model to each physiological data window to generate a prediction of the human state. In one embodiment, the prediction model may be an ML model that utilizes features extracted from the physiological data, such as heart rate variability, brainwave patterns, or skin conductance levels, to predict the state. The model may employ algorithms such as support vector machines, neural networks, or decision trees to perform the mapping. In another embodiment, the system may utilize AI techniques, such as deep learning, to map complex patterns within the physiological data to the human state predictions, thereby enabling the detection of subtle and nuanced manifestations of the target state.

At operation 606, the system determines a loss for the plurality of human state predictions based on a loss function and the ground truth label. The loss function quantifies the error or discrepancy between the predictions generated by the model and the ground truth labels. In one embodiment, the loss function may be a mean squared error function, which measures the average squared difference between the predicted values and the ground truth human labels. In another embodiment, the loss function may be a cross-entropy loss function, which is particularly suitable for classification tasks where the output is a probability distribution over different states.

Transitioning to operation 608, the system updates the parameters of the human state prediction model based on the determined loss. The updating of the model's parameters is an iterative process that involves adjusting the weights and biases within the model to reduce the loss. In one embodiment, the system may employ optimization algorithms such as gradient descent or stochastic gradient descent to perform the updates. These algorithms calculate the gradient of the loss function with respect to the model's parameters and make adjustments in the direction that minimizes the loss. In another embodiment, the system may utilize regularization techniques, such as L1 or L2 regularization, to prevent overfitting and ensure that the model generalizes well to new data.

At operation 610, the system determines the human state discriminatory power of the human state prediction model. This determination involves evaluating the model's ability to differentiate between various human states. In one embodiment, the system compares predictions generated for a first plurality of physiological data windows acquired while a first human state was induced in the subject with predictions generated for a second plurality of physiological data windows acquired while a second human state was induced in the subject. This comparison assesses the model's sensitivity and specificity in distinguishing between the states. In another embodiment, the system may validate the updated human state prediction model by comparing a set of human state predictions against a separate validation set of physiological data windows and associated ground truth labels. The validation set may be derived from a different continuous recording session of physiological data from the subject or a different subject, ensuring that the model's discriminatory power is robust across different subjects and conditions.

By leveraging extended-duration physiological recordings as ground truth, method 600 addresses the dynamic and variable nature of physiological manifestations that challenge the accurate detection of human states, such as cognitive load and drowsiness. The method's systematic segmentation of these recordings into shorter analysis windows, each labeled with ground truth indicative of the target human state, enables the development of models that are sensitive to the nuanced expressions of human states and robust across variable manifestations. Moreover, method 600 evaluates the model's discriminatory power, through the comparison of predictions across different induced states, to ensure that the models are capable of distinguishing between various human states, thereby enhancing their applicability in real-time monitoring scenarios. Method 600 thus represents a significant advancement in the continuous monitoring of human states, providing a foundation for the development of accurate and reliable human state prediction models that may be advantageously employed in applications requiring high-frequency state detection and analysis.

Referring to FIG. 7, a flowchart of a method 700 for evaluating the discriminatory power of a human state prediction model is shown. The method 700 utilizes a series of operations to process the physiological data, generate state predictions, and assess the model's ability to differentiate between various human states, which may be beneficial when developing continuous state prediction models, e.g., for comparing predictive models trained using different hyper parameters or training data.

At operation 702, the system maps each of a first plurality of physiological data windows acquired while a first human state was induced in a subject to a first plurality of human state predictions using a human state prediction model. The first human state may be one of several states, such as cognitive load or drowsiness, which are induced in the subject through various experimental setups designed to elicit specific human responses. In one embodiment, the system administers a cognitive task to the subject, such as an n-back task, a simulated driving task, or a pattern recognition task, to induce the first human state. The physiological data windows are segments of physiological data that have been previously recorded over a continuous duration of at least five minutes. In another embodiment, the physiological data includes at least one of ECG data, EEG data, PPG data, skin conductance data, and eye gaze data. Each analysis window is of a pre-determined duration, such as 30 seconds, and is designed to capture the dynamic and variable nature of physiological manifestations of the target human state.

Proceeding to operation 704, the system maps each of a second plurality of physiological data windows acquired while a second human state was induced in a subject to a second plurality of human state predictions using the human state prediction model. The second human state is distinct from the first and may represent a different level of cognitive load, a different type of stress, or a different stage of drowsiness, or a control where the first human state is absent (e.g., ensuring the subject is in a non-drowsy state, or under low cognitive load). In one embodiment, the system may adjust the cognitive task to induce the second human state, varying the complexity of the task, the frequency of task stimuli, or the duration for which the task is performed by the subject. In another embodiment, the system may employ a combination of sensory stimuli, such as auditory or visual cues, to induce the second human state, thereby simulating real-world conditions that may affect the subject's state, such as driving or operating machinery.

At operation 706, the system determines the human state discriminatory power of the human state prediction model based on the first plurality of human state predictions and the second plurality of human state predictions. This determination involves evaluating the model's ability to differentiate between the first and second human states. In one embodiment, the system compares predictions generated for the first plurality of physiological data windows with predictions generated for the second plurality of physiological data windows to assess the model's accuracy and sensitivity to state variations. As will be discussed in more detail in the below description of FIG. 8, the discriminatory power of the human state prediction model may be determined according to a first embodiment where an average is determined for the first plurality of predictions generated for the first human state, and compared to an average determined for the second plurality of predictions generated for the second human state, as depicted in many-to-many comparison 800B shown in FIG. 8. In another embodiment, a first prediction generated for the first human state may be compared to each of the plurality of predictions generated for the second human state, to produce an average difference between the first prediction and the plurality of predictions of the second human state. This procedure may be repeated for each of the first plurality of predictions generated for the first human state, producing a one-to-many comparison for each of the first plurality of human state predictions, as depicted in one-to-many comparison 800A shown in FIG. 8.

In this way, method 700 evaluates the model's discriminatory power, through the comparison of predictions across different induced states, to ensure that the models are capable of distinguishing between various human states, thereby enhancing their applicability in real-time monitoring scenarios.

Referring to FIG. 8, a graphical depiction of human data comparisons is shown, illustrating both one-to-many and many-to-many comparison approaches for evaluating the discriminatory power of a human state prediction model. The figure is divided into two main sections, one-to-many comparison 800A and many-to-many comparison 800B, each demonstrating a method for comparing human state predictions to assess the model's accuracy and sensitivity to state variations.

In one-to-many comparison 800A, a first human state 820 is represented by a series of windows, including window 804, window 806, and window 808. Each window corresponds to a shorter analysis window derived from a continuous recording of physiological data, as described herein. These windows capture the dynamic and variable nature of physiological manifestations associated with the first human state 820, which may include states such as cognitive load or drowsiness during activities like driving.

In one embodiment, window 804 may represent a 30-second segment of physiological data where the subject is experiencing a high cognitive load due to a complex driving simulation task. window 806 and window 808 may represent subsequent 30-second segments capturing different aspects of the high cognitive load state, such as decision-making and response reflection, respectively. These windows are analyzed to generate human state predictions, which are then compared to a second human state 840, represented by window 810, window 812, and window 814. The second human state 840 may reflect a different level of cognitive load or a control state with minimal cognitive demands.

In another embodiment, the one-to-many comparison 800A may involve comparing a first prediction generated for the first human state 820 against each of the plurality of predictions generated for the second human state 840. This comparison assesses the model's ability to distinguish between the two states based on the physiological data captured in the respective windows.

Many-to-many comparison 800B extends the comparison approach by incorporating averages of predictions for both the first and second human states. First average 850 represents the average of predictions for window 804, window 806, and window 808, which collectively characterize the first human state 820. This average serves as a representative measure of the model's predictions for the first state, capturing the overall trend of the physiological manifestations over the selected time frame.

In one embodiment, first average 850 may be calculated by averaging the output probabilities or categorical predictions generated by the human state prediction model for each of the windows associated with the first human state 820. This average provides a consolidated view of the model's performance across multiple segments of physiological data, reflecting the composite effect of various manifestations of the target state.

Second average 860, on the other hand, represents the average of predictions for window 810, window 812, and window 814, which collectively characterize the second human state 840. In a similar embodiment, second average 860 may be determined by averaging the model's predictions for each window associated with the second state, providing a comparable measure to first average 850.

In another embodiment, the many-to-many comparison 800B may involve calculating the average difference between first average 850 and second average 860 to assess the discriminatory power of the human state prediction model. This comparison may reveal the model's ability to differentiate between the first and second human states based on the aggregated predictions for each state. The comparison may also take into account the variability within each set of windows, providing insights into the model's consistency and reliability in state prediction.

The comparisons depicted in FIG. 8 may be applied to various human states beyond cognitive load and drowsiness, such as stress or fatigue, and may be utilized in different application domains, including automotive safety, healthcare monitoring, and workplace ergonomics.

Referring to FIG. 9, a flowchart of a method 900 for evaluating the performance of a mathematical model in predicting physiological states from continuous physiological data recordings is shown. The method 900 enables assessing the accuracy and reliability of a mathematical model in the context of human state detection. Method 900 advantageously consolidates a plurality of predictions made over shorter duration physiological data windows into an aggregate performance metric which better represents the mathematical models predictive ability over a continuous physiological data recording, while still enabling the mathematical model to make human state predictions for shorter duration physiological data windows.

Method 900 begins at operation 902, where the system selects a plurality of physiological data windows derived from a continuous physiological data recording, along with a corresponding ground truth state label. The continuous physiological data recording is obtained over a duration equal to or greater than a threshold duration, ensuring that a broad spectrum of physiological manifestations of the target state is captured. Each physiological data window is of a pre-determined duration, and less than the threshold duration, allowing for the continuous monitoring of the subject's state with enhanced temporal resolution. In another embodiment, the ground truth state label is indicative of the target state, such as cognitive load or drowsiness, which the subject was intended to experience during the data acquisition phase.

Proceeding to operation 904, the system predicts a state for each of the plurality of physiological data windows using a mathematical model. The mathematical model is configured to map the physiological data windows to corresponding state predictions. In one embodiment, the mathematical model may be a machine learning model that utilizes features extracted from the physiological data to predict the state. The model may employ algorithms such as support vector machines, neural networks, or decision trees to perform the predictions. In another embodiment, the system may utilize artificial intelligence techniques, such as deep learning, to map complex patterns within the physiological data to the state predictions, thereby enabling the detection of subtle and nuanced manifestations of the target state.

In one embodiment, the mathematical model may be a supervised machine learning model that has been trained on a dataset comprising physiological data windows and corresponding ground truth state labels. The model may use a variety of features extracted from the physiological data, such as heart rate variability, brainwave patterns, or skin conductance levels, to make its predictions. The features may be selected based on their relevance to the target state and their ability to discriminate between different states. For example, features that are strongly correlated with cognitive load may be used to predict states of high cognitive demand.

In another embodiment, mathematical models that do not rely on machine learning or deep learning, such as statistical models or rule-based systems, may be utilized to predict states based on the physiological data. These models may employ predefined rules, statistical analyses, or mathematical equations to infer the target state from the physiological data. For example, a statistical model may use regression analysis to determine the relationship between physiological measures and the target state, while a rule-based system may apply a set of expert-defined rules to classify the physiological data into different states.

At operation 906, the system determines a plurality of performance metrics for the mathematical model, one for each of the state predictions, using the ground truth state label. These performance metrics quantify the accuracy of the model's predictions in comparison to the ground truth. In one embodiment, the performance metrics may include measures such as precision, recall, F1 score, and accuracy. In another embodiment, the system may calculate a confusion matrix to visualize the performance of the model across different states, providing insights into the model's sensitivity and specificity.

At operation 908, the system aggregates the plurality of performance metrics to produce an aggregate performance metric for the continuous physiological data recording. This aggregation provides a consolidated view of the model's overall performance. In one embodiment, the aggregate performance metric may be a weighted average of the individual performance metrics, taking into account the relative importance of each metric. In another embodiment, the system may employ statistical methods to combine the performance metrics, such as calculating the area under the receiver operating characteristic (ROC) curve or the precision-recall curve, which are indicative of the model's discriminative ability.

At operation 910, the system stores the aggregate performance metric in non-transitory memory. The storage of the aggregate performance metric facilitates the later retrieval and utilization of the data for further analysis or model refinement. In one embodiment, the system organizes the stored data in a structured database, allowing for efficient querying and access to the performance metrics and associated labels. Following operation 910, method 900 may end.

The method 900 enables the generation of robust performance evaluations for mathematical models used in human state detection. Method 900 systematically consolidates predictions from segmented physiological data windows into an aggregate performance metric, thereby providing a quantitative assessment of the mathematical model's predictive accuracy over extended physiological recordings. This method ensures that an accurate performance metric is determined for the model while ensuring the model retains its predictive functionality for shorter duration data windows, facilitating robust human state detection across varying temporal scales.

The disclosure also provides support for a method comprising: recording physiological data from a subject in a target state over a continuous duration equal to or greater than a threshold duration, segmenting the recorded physiological data into a plurality of shorter analysis windows, each analysis window being of a pre-determined duration of less than the threshold duration, labeling the plurality of shorter analysis windows with a ground truth label indicative of the target state, and storing the plurality of shorter analysis windows and the ground truth label in non-transitory memory. In a first example of the method the method further comprising: inducing the target state in the subject by: administering one or more of a cognitive task, an audio-visual stimuli, and a environmental factors to elicit the target state, and adjusting a level of the target state by varying a complexity of the cognitive task, adjusting an intensity of the audio-visual stimuli, or adjusting an intensity of the stressogenic factor. In a second example of the method, optionally including the first example, the physiological data includes at least one of electrocardiogram (ECG) data, electroencephalogram (EEG) data, photoplethysmogram (PPG) data, skin conductance data, and eye gaze data. In a third example of the method, optionally including one or both of the first and second examples the method further comprising: detecting in each of the plurality of shorter analysis windows occurrence of one or more of a pre-determined set of manifestations of the target state, and labeling each of the plurality of shorter analysis windows with a secondary ground truth label indicative of the detected manifestations of the target state. In a fourth example of the method, optionally including one or more or each of the first through third examples the method further comprising: filtering the plurality of shorter analysis windows based on a plurality of respective secondary ground truth labels indicative of manifestations of the target state. In a fifth example of the method, optionally including one or more or each of the first through fourth examples, segmenting the recorded physiological data into the plurality of shorter analysis windows includes selecting a window size and splitting the recorded physiological data into consecutive analysis windows by a predetermined step duration of less than the pre-determined duration of the plurality of shorter analysis windows. In a sixth example of the method, optionally including one or more or each of the first through fifth examples, the method further comprises: predicting a state for each of the plurality of shorter analysis windows using a mathematical model, wherein the mathematical model is configured to map the plurality of shorter analysis windows to a plurality of state predictions. In a seventh example of the method, optionally including one or more or each of the first through sixth examples, the method further comprises: determining a plurality of performance metrics for the mathematical model, one for each of the plurality of state predictions, using the ground truth label, wherein the plurality of performance metrics include at least one of precision, recall, F1 score, and accuracy, and aggregating the plurality of performance metrics to produce an aggregate performance metric for the recorded physiological data, wherein the aggregate performance metric is a weighted average of the plurality of performance metrics.

The disclosure also provides support for a system for inducing and evaluating a target state in a subject, the system comprising: a processor, and a non-transitory memory storing instructions that, when executed by the processor, cause the system to: induce the target state in the subject, record physiological data from the subject over a continuous duration equal to or greater than a threshold duration, segment the recorded physiological data into a plurality of shorter analysis windows, each analysis window being of a pre-determined duration of equal to or less than the threshold duration, label each of the plurality of shorter analysis windows with a ground truth label indicative of the target state, and store the plurality of shorter analysis windows and the ground truth label in the non-transitory memory. In a first example of the system, the processor is further configured to: administer a cognitive task to the subject to elicit the target state, the cognitive task comprising one of an n-back task, a simulated driving task, and a pattern recognition task, and adjust a level of cognitive load experienced by the subject by varying a complexity of the cognitive task, a frequency of task stimuli, and a duration for which the cognitive task is performed by the subject. In a second example of the system, optionally including the first example, the processor is further configured to: detect in each of the plurality of shorter analysis windows occurrence of one or more of a pre-determined set of manifestations of the target state, and label each of the plurality of shorter analysis windows with a secondary ground truth label indicative of the detected manifestations of the target state. In a third example of the system, optionally including one or both of the first and second examples, the processor is further configured to: filter the plurality of shorter analysis windows based on a plurality of respective secondary ground truth labels indicative of manifestations of the target state. In a fourth example of the system, optionally including one or more or each of the first through third examples, the pre-determined duration of the plurality of shorter analysis windows is 30 seconds. In a fifth example of the system, optionally including one or more or each of the first through fourth examples, the processor is further configured to: segment the recorded physiological data into the plurality of shorter analysis windows by overlapping consecutive analysis windows by a predetermined step duration of less than the pre-determined duration of the plurality of shorter analysis windows.

The disclosure also provides support for a method for training a state prediction model, comprising: inducing a target state in a subject, recording physiological data from the subject over a continuous duration greater than or equal to a threshold duration, segmenting the recorded physiological data into a plurality of physiological data windows, each of the plurality of physiological data windows being of a pre-determined duration of less than the threshold duration, labeling each of the plurality of physiological data windows with a ground truth label indicative of the target state, storing the plurality of physiological data windows and the ground truth label in non-transitory memory, selecting a training data pair, comprising the plurality of physiological data windows of pre-determined duration, and the ground truth label indicative of the target state, mapping the plurality of physiological data windows to a corresponding plurality of state predictions using the state prediction model, determining a loss for the plurality of state predictions based on a loss function and the ground truth label, and updating parameters of the state prediction model based on the determined loss. In a first example of the method, selecting the training data pair further comprises filtering the plurality of physiological data windows based on secondary ground truth labels associated with detected manifestations of the target state. In a second example of the method, optionally including the first example, recording physiological data from the subject further comprises utilizing at least one physiological data acquisition device configured to measure one or more of heart rate, skin conductance, brain activity, and respiratory rate. In a third example of the method, optionally including one or both of the first and second examples, the method further comprises: determining a state discriminatory power of the state prediction model by comparing predictions generated for a first plurality of physiological data windows acquired while a first state was induced in the subject with predictions generated for a second plurality of physiological data windows acquired while a second state was induced in the subject. In a fourth example of the method, optionally including one or more or each of the first through third examples, the method further comprises: validating the updated state prediction model by comparing a set of state predictions against a separate validation set of data windows and associated ground truth labels. In a fifth example of the method, optionally including one or more or each of the first through fourth examples, the separate validation set of physiological data windows is derived from a different continuous recording session of data from the subject or a different subject.

Aspects of the present disclosure are described above with reference to flowchart illustrations and/or block diagrams of methods, apparatus (systems) and computer program products according to embodiments of the disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, enable the implementation of the functions/acts specified in the flowchart and/or block diagram block or blocks. Such processors may be, without limitation, general purpose processors, special-purpose processors, application-specific processors, or field-programmable processors.

While the foregoing is directed to embodiments of the present disclosure, other and further embodiments of the disclosure may be devised without departing from the basic scope thereof, and the scope thereof is determined by the claims that follow.

## Claims

1. A method comprising:
recording physiological data from a subject in a target state over a continuous duration equal to or greater than a threshold duration;
segmenting the recorded physiological data into a plurality of shorter analysis windows, each analysis window being of a pre-determined duration of less than the threshold duration;
labeling the plurality of shorter analysis windows with a ground truth label indicative of the target state; and
storing the plurality of shorter analysis windows and the ground truth label in non-transitory memory.

2. The method of claim 1, the method further comprising:
inducing the target state in the subject by:
administering one or more of a cognitive task, and an audio-visual stimuli to elicit the target state; and
adjusting a level of the target state by varying a complexity of the cognitive task, or adjusting an intensity of the audio-visual stimuli.

3. The method of claim 1, wherein the physiological data includes at least one of electrocardiogram (ECG) data, electroencephalogram (EEG) data, photoplethysmogram (PPG) data, skin conductance data, and eye gaze data.

4. The method of claim 1, the method further comprising:
detecting in each of the plurality of shorter analysis windows occurrence of one or more of a pre-determined set of manifestations of the target state; and
labeling each of the plurality of shorter analysis windows with a secondary ground truth label indicative of the detected manifestations of the target state.

5. The method of claim 4, the method further comprising:
filtering the plurality of shorter analysis windows based on a plurality of respective secondary ground truth labels indicative of manifestations of the target state.

6. The method of claim 1, wherein segmenting the recorded physiological data into the plurality of shorter analysis windows includes selecting a window size and splitting the recorded physiological data into consecutive analysis windows by a predetermined step duration of less than the pre-determined duration of the plurality of shorter analysis windows.

7. The method of claim 1, further comprising:
predicting a state for each of the plurality of shorter analysis windows using a mathematical model, wherein the mathematical model is configured to map the plurality of shorter analysis windows to a plurality of state predictions.

8. The method of claim 7, further comprising:
determining a plurality of performance metrics for the mathematical model, one for each of the plurality of state predictions, using the ground truth label, wherein the plurality of performance metrics include at least one of precision, recall, F1 score, and accuracy; and
aggregating the plurality of performance metrics to produce an aggregate performance metric for the recorded physiological data, wherein the aggregate performance metric is a weighted average of the plurality of performance metrics.

9. A system for inducing and evaluating a target state in a subject, the system comprising:
a processor; and
a non-transitory memory storing instructions that, when executed by the processor, cause the system to:
induce the target state in the subject;
record physiological data from the subject over a continuous duration equal to or greater than a threshold duration;
segment the recorded physiological data into a plurality of shorter analysis windows, each analysis window being of a pre-determined duration of equal to or less than the threshold duration;
label each of the plurality of shorter analysis windows with a ground truth label indicative of the target state; and
store the plurality of shorter analysis windows and the ground truth label in the non-transitory memory.

10. The system of claim 9, wherein the processor is further configured to:
administer a cognitive task to the subject to elicit the target state, the cognitive task comprising one of an n-back task, a simulated driving task, and a pattern recognition task; and
adjust a level of cognitive load experienced by the subject by varying a complexity of the cognitive task, a frequency of task stimuli, and a duration for which the cognitive task is performed by the subject.

11. The system of claim 9, wherein the processor is further configured to:
detect in each of the plurality of shorter analysis windows occurrence of one or more of a pre-determined set of manifestations of the target state; and
label each of the plurality of shorter analysis windows with a secondary ground truth label indicative of the detected manifestations of the target state.

12. The system of claim 11, wherein the processor is further configured to:
filter the plurality of shorter analysis windows based on a plurality of respective secondary ground truth labels indicative of manifestations of the target state.

13. The system of claim 9, wherein the pre-determined duration of the plurality of shorter analysis windows is 30 seconds.

14. The system of claim 9, wherein the processor is further configured to:
segment the recorded physiological data into the plurality of shorter analysis windows by overlapping consecutive analysis windows by a predetermined step duration of less than the pre-determined duration of the plurality of shorter analysis windows.
